# EUROPEAN PATENT APPLICATION

(11) **EP 4 674 466 A1**
(43) Date of publication of application: **07.01.2026**
(21) Application number: 24796181.6
(22) Date of filing: 25.04.2024
(51) Int. Cl.: A61M 60/178

(54) **CATHETER PUMP**

(30) Priority: 26.04.2023 CN 202310459662
(71) Applicant: Magassist Co., Ltd., Suzhou, Jiangsu 215163 (CN)
(72) Inventor: TU, Pan, Suzhou, Jiangsu 215163 (CN); YEN, Ifan, Suzhou, Jiangsu 215163 (CN)
(74) Representative: Winter, Brandl - Partnerschaft mbB
(86) International application number: PCT/CN2024/089831
(87) International publication number: WO 2024/222805

(57) **Abstract**

Provided is a catheter pump, including a catheter, a proximal connecting portion and a pump head, wherein the proximal connecting portion is connected to a distal end of the catheter; the pump head includes a stent and an impeller accommodated in the stent, and the impeller is configured to be driven to rotate for blood pumping; the stent includes a stent main body and a plurality of proximal connecting legs located at an axial proximal end of the stent main body, and the plurality of proximal connecting legs are arranged at intervals along a circumferential direction of the stent; each of the proximal connecting legs includes a proximal connecting strut and a proximal supporting strut located at a distal end of the proximal connecting strut ; the proximal supporting strut is connected to the stent main body; the proximal connecting strut is fixedly connected to the proximal connecting portion.

## Description

This application claims priority to Patent Application No. 2023104596624, filed with the China National Intellectual Property Administration on April 26, 2023, and titled "Catheter Pump".

### Technical Field

The present disclosure relates to the field of medical devices, and particularly to a catheter pump.

### Background

Catheter pumps can be classified into non-collapsible types and collapsible types. Among them, the collapsible catheter pump results in a smaller surgical wound during intervention, thereby offering the advantage of more convenient and rapid application.

Moreover, as the collapsible catheter pump needs to be pre-collapsed to reduce the size of the pump head and then expanded and deployed after being delivered to a specific location (e.g., the left ventricle), a corresponding structure is required to form a pump housing. In consideration of simplifying the manufacturing process as much as possible, a single preformed tube can be used to fabricate a stent portion of the pump housing through engraving or laser cutting. Additionally, a connecting collar is provided at a proximal end of the stent to enable connection and fixation with a catheter, such that the diameter of the sleeve-shaped connecting collar is the same as that of the preformed tube used to form the stent portion.

Since the final product must meet the requirement of a small size for easy placement and intervention, the diameter of the preformed tube is relatively small. Meanwhile, to achieve a large final expanded diameter required for the stent portion, more material must be removed by engraving, resulting in a smaller strut width and lower stiffness of the stent. Conversely, if the strut width and stiffness of the stent meet the requirements, less material will be removed by engraving, which in turn leads to a smaller expanded diameter of the stent.

Additionally, a preformed tube with a larger diameter may also be engraved to fabricate the stent, and the connecting collar at the proximal end is then thinned. However, the approach results in material waste, higher cost, and a more complex process.

In fact, as in the known embodiment disclosed in CN114588533A, it is art known to inventors that a distal connecting structure of a stent adopts discrete legs (specifically, a plurality of legs arranged at circumferential intervals). However, to maintain a high-strength fixed connection with a catheter or a proximal bearing chamber, most of the current proximal connecting structures of the stents adopt a circumferentially continuous collar structure. The reason, which will be described in detail below, is mainly that the circumferentially continuous collar structure does not tilt due to the lever principle when the pump head is collapsed, thereby consistently maintaining a fixed connection with the catheter. In view of this, the proximal connecting structure of the stent cannot adopt the same or similar discrete leg structure as the distal connecting structure.

Further, the connecting structures at both ends of the stent are respectively connected to fixed components (for instance, which may be the proximal and distal bearing chambers, respectively). The connecting structures at the two ends have portions that are structurally connected to or overlapped with the fixed components, while the portions extending from the fixed components form cantilever structures. An inlet portion and an outlet portion of the stent need to be supported by the cantilever structures, while a main body portion for further accommodating an impeller is supported by the inlet portion and the outlet portion. Therefore, how the cantilever structures have better supporting stiffness is crucial to the overall stiffness during expansion of the stent.

In particular, the proximal connection structure, in some cases, for example, when collapsing the pump head from the proximal end, the proximal cantilever structure deforms when subjected to forced compression by the collapsed sheath. The compressive deformation must be recovered by the inherent elastic memory (the entire stent is made of a memory material) of the cantilever structure after the sheath is withdrawn, so as to support the expansion of the inlet portion and the main body portion. Therefore, it is also of vital importance to optimally maintain the supporting stiffness of the proximal cantilever structure.

### Summary

In view of the shortcomings of the art known to inventors, an objective of the present disclosure is to provide a catheter pump that ensures a stent main body has a large expanded diameter in a radially expanded state while meeting the requirement of a small size of the stent main body in a radially collapsed state, and that the stiffness of the stent main body meets the requirements. Meanwhile, it can also ensure that a cantilever structure included in the proximal connecting legs of a stent has favorable supporting stiffness. Furthermore, it can also ensure that a cantilever structure included in distal connecting legs of the stent has favorable supporting stiffness.

To achieve the above objective, the present disclosure provides the following solutions:
A catheter pump includesa catheter, a proximal connecting portion connected to a distal end of the catheter, and a pump head. The pump head includes a stent and an impeller accommodated in the stent, where the impeller is configured to be driven to rotate for blood pumping. The stent is operable to switch between a radially collapsed state and a radially expanded state. In the radially expanded state, the stent includes a stent main body and a plurality of proximal connecting legs located at a proximal end of the stent main body. The plurality of proximal connecting legs are arranged at intervals along a circumferential direction of the stent. Each of the proximal connecting legs includes a proximal connecting strut and a proximal supporting strut located at a distal end of the proximal connecting strut. The proximal supporting strut is connected to and supports the stent main body, and a circumferential width of the proximal supporting strut is greater than a circumferential width of the proximal connecting strut. The proximal connecting strut is fixedly connected to the proximal connecting portion. The proximal supporting strut includes a first connecting portion fixedly connected to the proximal connecting portion and a first outer side portion located outside a distal end of the proximal connecting portion.

Preferably, each of the proximal connecting legs further includes a proximal wide body portion fixedly connected to the proximal connecting portion, and a circumferential width of the proximal wide body portion is greater than a circumferential width of the proximal connecting strut.

Preferably, the proximal wide body portion is located at a proximal end of the proximal connecting strut.

Preferably, an axial length of the first connecting portion is greater than an axial length of the proximal wide body portion and smaller than an axial length of the proximal connecting strut.

Preferably, an outer wall of the proximal connecting portion is provided with a plurality of proximal leg grooves, in which the plurality of proximal connecting legs are snapped in a one-to-one correspondence, and a proximal collar is sleeved over the proximal connecting portion and configured to fix the proximal connecting legs in a radial direction of the stent so as to retain the proximal connecting legs within the proximal leg grooves.

Preferably, a proximal spacing protrusion is formed on the outer wall of the proximal connecting portion between two circumferentially adjacent proximal leg grooves, the proximal spacing protrusion includes a first protruding portion located between two adjacent first connecting portions and a second protruding portion located between two adjacent proximal connecting struts, and a circumferential width of the second protruding portion is greater than a circumferential width of the first protruding portion.

Preferably, the proximal collar is made of a metallic material.

Preferably, the circumferential width of the proximal connecting strut remains unchanged in an extending direction thereof.

Preferably, the circumferential width of the proximal supporting strut remains unchanged in an extending direction thereof.

Preferably, the circumferential width of the proximal wide body portion remains unchanged in the extending direction of the proximal connecting strut.

Preferably, a proximal transition portion is provided between the proximal supporting strut and the proximal connecting strut. In a direction from a proximal end of the proximal transition portion to a distal end of the proximal transition portion, a circumferential width of the proximal transition portion gradually increases from being equal to that of the proximal connecting strut to being equal to that of the proximal supporting strut.

Preferably, the proximal connecting portion is a proximal bearing chamber provided at the distal end of the catheter, a proximal bearing is disposed in the proximal bearing chamber, and a drive shaft passes through the proximal bearing and is connected to the impeller so as to drive the impeller to rotate.

Preferably, the stent further includes a plurality of distal connecting legs located at a distal end of the stent main body, and the plurality of proximal connecting legs are arranged at intervals along a circumferential direction of the stent. Each of the distal connecting legs includes a distal connecting strut and a distal supporting strut located at a proximal end of the distal connecting strut. The distal supporting strut is connected to and supports the stent main body, and a circumferential width of the distal supporting strut is greater than a circumferential width of the distal connecting strut. The distal connecting strut is fixedly connected to a distal connecting portion. The distal supporting strut includes a second connecting portion fixedly connected to the distal connecting portion and a second outer side portion located outside a proximal end of the distal connecting portion.

Preferably, each of the distal connecting legs further includes a distal wide body portion fixedly connected to the distal connecting portion, and a circumferential width of the distal wide body portion is greater than a circumferential width of the distal connecting strut.

Preferably, the distal wide body portion is located at a distal end of the distal connecting strut.

Preferably, an axial length of the second connecting portion is greater than an axial length of the distal wide body portion and smaller than an axial length of the distal connecting strut.

Preferably, an outer wall of the distal connecting portion is provided with a plurality of distal leg grooves, in which the plurality of distal connecting legs are snapped in a one-to-one correspondence, and a distal collar is sleeved over the distal connecting portion and configured to fix the distal connecting legs in the radial direction of the stent so as to retain the distal connecting legs within the distal leg grooves.

Preferably, a distal spacing protrusion is formed on the outer wall of the distal connecting portion between two circumferentially adjacent distal leg grooves, the distal spacing protrusion includes a fourth protruding portion located between two adjacent second connecting portions and a fifth protruding portion located between two adjacent distal connecting struts, and a circumferential width of the fifth protruding portion is greater than a circumferential width of the fourth protruding portion.

Preferably, the distal collar is made of a metallic material.

Preferably, the circumferential width of the distal connecting strut remains unchanged in an extending direction thereof.

Preferably, the circumferential width of the distal supporting strut remains unchanged in an extending direction thereof.

Preferably, the circumferential width of the distal wide body portion remains unchanged in the extending direction of the distal connecting strut.

Preferably, a distal transition portion is provided between the distal supporting strut and the distal connecting strut. In a direction from a distal end of the distal transition portion to a proximal end of the distal transition portion, a circumferential width of the distal transition portion gradually increases from being equal to that of the distal connecting strut to being equal to that of the distal supporting strut.

Preferably, the distal connecting portion is a distal bearing chamber connected to a distal end of the stent, a distal bearing is disposed in the distal bearing chamber, and a drive shaft passes through the distal bearing and is connected to the impeller so as to drive the impeller to rotate.

Preferably, the distal connecting portion is a protective tip connected to a distal end of the distal connecting legs, and the protective tip is flexible so as to separate a blood inlet of the pump head from a ventricular wall.

Preferably, a hydrophobic coating is provided on a surface of the stent.

Preferably, a hydrophobic coating is provided on a surface of the stent main body.

Preferably, the hydrophobic coating is a superhydrophobic coating.

In the catheter pump provided in the present embodiment, the plurality of proximal connecting legs are arranged at intervals in the circumferential direction of the stent, i.e., the plurality of proximal connecting legs are arranged discretely, thereby replacing a connecting collar with an at least partially circumferentially continuous structure in the prior art. As mentioned above, if a circumferentially continuous connecting collar structure is adopted (the connecting collar is a proximal portion of the preformed tube), the diameter of the connecting collar is the diameter of the collapsed stent. In other words, the diameter of the collapsed stent is limited by the diameter of the connecting collar at the proximal end, that is, limited by the diameter of the preformed tube. If the diameter of the preformed tube is relatively large, it is difficult to achieve a small diameter of the collapsed stent, and thus, the requirement for a small interventional size of the pump head cannot be met. If the diameter of the preformed tube is relatively small, although it can meet the requirements for the small collapsed size and small interventional size of the stent, and cannot simultaneously satisfy the requirements for a large expanded diameter and high expanded supporting stiffness of the stent. The reason is as follows: To achieve a large expanded diameter, more material needs to be cut and removed from the preformed tube, which results in a small width of a strut of the stent, especially the main body portion, thus leading to a decrease in the supporting stiffness after expansion. Conversely, to achieve high expanded supporting stiffness, the strut of the stent, especially the stent main body, is required to have a relatively large width. Thus, not too much material needs to be cut and removed from the preformed tube, but this, in turn, results in the stent having an insufficiently large expanded diameter.

The technical effect of the expanded stent having high supporting stiffness is described in detail below and will not be repeated here. In contrast, a large expanded diameter of the stent allows for an increased diameter of the impeller, thereby achieving the technical effect of increasing the blood pumping flow rate.

On the other hand, the stent of the present disclosure no longer adopts a circumferentially continuous collar structure at the proximal end, and instead, adopts a plurality of discrete leg-like structures that are not connected to each other. In this way, the diameter of the collapsed stent is not limited by the diameter of the preformed tube, which means that a preformed tube with a relatively larger diameter can be used for manufacturing the stent by laser cutting. Since the diameter of the selected preformed tube is larger than that in the prior art, to achieve the same expanded diameter, the amount of material cut and removed is reduced, and the width of the struts in the main body portion of the stent is increased, thereby improving the supporting stiffness of the stent. Alternatively, to achieve the same supporting stiffness, the amount of material cut and removed can be increased, and the width of the struts in the main body portion of the stent is reduced, thereby allowing for an increased expanded diameter of the stent.

It is worth noting that the stent of the present disclosure is fabricated as a whole from a preformed tube by laser cutting. That is, compared with the prior art in which only the main body portion and distal connecting legs of the stent are formed by laser cutting, the present disclosure uses laser cutting to form all structures of the stent, including the main body portion, the proximal connecting legs, and the distal connecting legs. In this way, the manufacturing process of the stent is instead simplified.

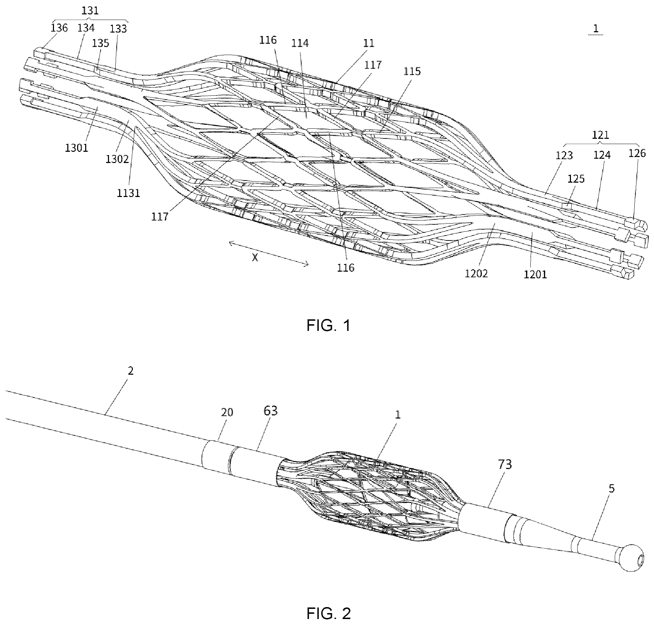

The stent fabricated in the above manner has a hollow cylindrical structure (at this point, portions such as the main body portion, the proximal connecting legs, and the distal connecting legs are not distinguished), and the outer diameter is consistent across all axial portions. Subsequently, the hollow cylindrical structure (referred to as the "stent before shaping" for short) is subjected to a shaping process to obtain a final stent structure. Specifically, the stent before shaping is sleeved over an inner shaping mold, and then an outer shaping mold is sleeved over the inner shaping mold (the outer contour shape of the inner shaping mold and the shape of an inner cavity of the outer shaping mold can refer to the stent shape as shown in FIG. 1). Subsequently, a heat treatment process is performed on the stent before shaping. The stent is heated to a phase transition temperature of a stent material (e.g., nickel-titanium alloy), held at the temperature for a certain period of time, then cooled, and demolded to obtain the final stent.

Therefore, compared with the prior art, the stent of the present disclosure adopts a structure with discrete proximal connecting legs, which can not only ensure that the stent main body has a small size when in the radially collapsed state, but also guarantee that the stent main body has a relatively large expanded diameter and high supporting stiffness when in the radially expanded state. Furthermore, compared with manufacturing a stent with a connecting collar at the proximal end by engraving a preformed tube with a relatively large diameter, the present disclosure produces no waste material, resulting in lower costs and a simpler process.

Furthermore, a portion of the proximal supporting strut (the first connecting portion) with a larger width in the proximal connecting leg is fixedly connected to the proximal connecting portion, which can provide support for the first outer side portion with a cantilever structure extending to the outside of the proximal connecting portion. This ensures that a proximal cantilever structure of the stent has favorable supporting stiffness (avoiding supporting the cantilever structure via the proximal connecting strut with a smaller width), thereby enabling the proximal cantilever structure of the stent to possess excellent supporting stiffness. In this way, only when the main body portion of the stent is supported by a cantilever portion with high supporting stiffness can the main body portion of the stent achieve high supporting stiffness in the expanded state.

As mentioned above, a portion of the proximal supporting strut (the first connecting portion) is fixedly connected to the proximal connecting portion, while the other portion (the first outer side portion) extends outward from the distal end of the proximal connecting portion. This also means that the proximal supporting strut spans the proximal connecting portion. In this case, during the collapsing or expanding process, with the distal end of the proximal connecting portion as the boundary, the junction between a deformable section and a non-deformable section of the stent is located at the proximal supporting strut. It should be known to those skilled in the art that the deformation junction is most prone to stress concentration and stress fatigue, which will lead to a decrease in material stiffness. Therefore, by increasing the width of the proximal supporting strut, the stiffness of the proximal supporting strut is positively compensated, thereby preventing the proximal supporting strut from potential fracture caused by repeated collapsing or expanding of the stent.

In contrast, the proximal connecting strut with a smaller width mainly functions to achieve axial positioning and fixed connection with the proximal connecting portion, ensuring that the proximal connecting legs are more firmly connected to the proximal connecting portion and preventing connection failure between the proximal connecting legs and the proximal connecting portion.

That is, the proximal connecting strut with a smaller circumferential width can flexibly and conveniently achieve connection with the proximal connecting portion. In addition, the proximal connecting portion, by connecting with the first connecting portion (which has a larger circumferential width), provides favorable stiffness support for the first outer side portion with a cantilever structure extending to the outside of the distal end of the proximal connecting portion, thereby ensuring that the main body portion of the stent has excellent supporting stiffness. Moreover, the excellent supporting stiffness of the stent main body is crucial for the stable operation of the pump head. For example, it can prevent the pump head from caving in when subjected to lateral impact inside the ventricle (i.e., when the stent is subjected to lateral force), thereby avoiding the impeller from touching the stent and preventing the impeller from being forced to stop rotating (which would otherwise result in blood pumping failure).

On the other hand, the proximal connecting strut with a smaller circumferential width has a longer axial length, which can increase a direct connection length between the proximal connecting legs and the proximal connecting portion. Furthermore, the proximal connecting portion can provide a certain strength support for the proximal connecting strut, thereby ensuring the connection strength between the stent and the proximal connecting portion.

The proximal wide body portion with a larger width is connected to the proximal connecting strut, and the outer wall of the proximal connecting portion is recessed inward to form proximal leg grooves for receiving the proximal connecting legs. Both the proximal wide body portion and the proximal connecting strut are fully snapped in the proximal leg grooves. In this way, the proximal wide body portion, the proximal connecting strut, and the proximal connecting portion form a hook-type physical connection structure. This hook-type connection structure can continuously resist the axial pulling force between the catheter and the stent, especially in scenarios where the pump head is collapsed from the proximal end of the stent. The effect of continuously resisting axial pulling force can stably maintain the connection between the catheter and the stent, preventing disconnection.

By increasing the length of the proximal connecting strut (with a smaller width), on the one hand, an axial connection length (overlap length) between the proximal connecting legs and the proximal connecting portion is increased to ensure the connection strength between the two. On the other hand, it can reduce the grooving area of the proximal connecting portion, ensuring that the structural strength of the proximal connecting portion is not excessively compromised. This, in turn, provides good structural support for the proximal cantilever structure of the stent.

On the other hand, the distal connecting legs formed at the distal end of the stent adopt the same structural design (mirror-symmetrical) as the proximal connecting legs, which ensures that a distal cantilever structure of the stent also has favorable supporting stiffness. In this way, the stent also obtains strong stiffness support from the distal end. Combined with the identical structure of the proximal connecting legs, the stent has favorable stiffness support effects at both ends, thereby ensuring the main body portion of the stent has excellent supporting stiffness in the expanded state.

The distal connecting legs adopt the same or similar structural design as the proximal connecting legs, and thus can achieve substantially the same technical effects. For details, reference can be made to the above description, which will not be repeated here.

### Brief Description of the Drawings

FIG. 1 illustrates a schematic structural diagram of a stent according to an embodiment of the present disclosure;
FIG. 2 illustrates a schematic structural diagram of a catheter pump according to an embodiment of the present disclosure;
FIG. 3 illustrates a schematic structural diagram of the connection between a proximal bearing chamber and proximal connecting legs according to an embodiment of the present disclosure;
FIG. 4 illustrates a schematic structural diagram of the connection between a distal bearing chamber and the distal connecting legs according to an embodiment of the present disclosure;
FIG. 5 illustrates a graph of experimental data of a stent provided with a hydrophobic coating according to an embodiment of the present disclosure;
FIG. 6 illustrates a schematic structural diagram of a proximal bearing chamber according to an embodiment of the present disclosure;
FIG. 7 illustrates a schematic structural diagram of the assembly of a distal bearing chamber and a protective tip according to an embodiment of the present disclosure;
FIG. 8 illustrates a schematic structural diagram of a catheter and a first connecting portion at the distal end thereof according to an embodiment of the present disclosure.

### Description of reference numerals:

1, stent; 2, catheter; 21, first connecting portion; 22, stop step; 20, heat-shrink tubing; 5, protective tip; 51, distal leg groove; 511, axial groove portion; 512, distal circumferential annular groove; 6, proximal bearing chamber; 61, proximal leg groove; 62, proximal spacing protrusion; 621, first protruding portion; 622, second protruding portion; 623, third protruding portion; 63, proximal collar; 67, first portion; 671, through hole; 68, second portion; 681, recess; 7, distal bearing chamber; 72, distal spacing protrusion; 722, fifth protruding portion; 723, fourth protruding portion; 73, distal collar; 11, stent main body; 1131, bifurcation structure; 114, mesh hole; 115, strut; 116, first strut; 117, second strut; 121, distal connecting leg; 123, distal supporting strut; 124, distal connecting strut; 125, distal transition portion; 126, distal wide body portion; 1201, second connecting portion; 1202, second outer side portion; 131, proximal connecting leg; 133, proximal supporting strut; 134, proximal connecting strut; 135, proximal transition portion; 136, proximal wide body portion; 1301, first connecting portion; 1302, first outer side portion.

### Detailed Description of the Embodiments

The present disclosure will be described in detail below with reference to specific embodiments shown in the accompanying drawings. However, these embodiments do not limit the present disclosure, and any structural, methodological, or functional modifications made by those of ordinary skill in the art based on these embodiments shall fall within the scope of protection of the present disclosure.

The terms "proximal", "distal", "rear", and "front" used in the present disclosure are defined relative to the clinician who operates the catheter pump of the present embodiment. The terms "proximal" and "rear" refer to the parts relatively close to the clinician, while the terms "distal" and "front" refer to the parts relatively far from the clinician. For example, an in-vitro part is located at the proximal end and the rear end, and an interventional in-vivo part is located at the distal end and the front end.

It should be understood that the orientations such as "proximal", "distal", "rear", and "front" are defined for the convenience of description. However, the catheter pump can be used in many orientations and positions; therefore, these terms describing relative positional relationships are not restrictive or absolute. For example, the above definitions of various orientations are only for the convenience of explaining the technical solution of the present disclosure, and do not limit the orientation of the catheter pump of the present disclosure in scenarios that may cause the catheter pump of the present disclosure to be inverted or the position of the catheter pump of the present disclosure to change, including but not limited to product testing, transportation, and manufacturing. In the present disclosure, if the above definitions are otherwise clearly specified and limited, they shall comply with such explicit specifications and limitations.

In the present disclosure, unless otherwise clearly specified and limited, terms such as "connected" and "connection" shall be understood in a broad sense. For example, the "connection" may be a fixed connection, a detachable connection, or a movable connection, or may be integrated into a single unit; it may be a direct connection, or an indirect connection through an intermediate medium, and may also refer to the communication between the interiors of two components or the interaction relationship between two components. The specific meanings of the above terms in the present disclosure may be understood by those of ordinary skill in the art depending on specific circumstances.

In addition, the technical features involved in the various embodiments of the present disclosure described below can be combined with each other as long as they do not conflict with one another.

The catheter pump according to the embodiments of the present disclosure is configured to perform a partial blood pumping function of the heart. In a scenario applicable to left ventricular assistance, the catheter pump pumps blood from the left ventricle into the aorta, providing support for blood circulation, reducing the workload of the heart of a subject, or providing additional continuous blood pumping power support when the heart pumping capacity is insufficient. Of course, the catheter pump can also be introduced into other target positions of the subject as desired by means of interventional surgery, such as the right ventricle, blood vessels, or other organs.

Please refer to FIGs. 1 to 4 and FIGs. 6 to 8. A catheter pump according to the embodiment of the present disclosure includes a catheter 2, a proximal connecting portion, and a pump head. The proximal connecting portion is connected to a distal end of the catheter 2. The pump head includes a stent 1 and an impeller accommodated in the stent 1, and the impeller is configured to be driven to rotate for blood pumping. The stent 1 is operable to switch between a radially collapsed state and a radially expanded state. In the radially expanded state, the stent 1 includes a stent main body 11 and proximal connecting legs 131 located at a proximal end of the stent main body 11. A plurality of proximal connecting legs 131 are provided. The plurality of proximal connecting legs 131 are arranged at intervals along a circumferential direction of the stent 1. The proximal connecting legs 131 and the stent main body 11 are of an integrated structure.

It should be noted that two ends of the catheter 2 are a proximal end and a distal end, respectively; the stent main body 11 has two opposite ends, and the two opposite ends of the stent main body 11 are a proximal end and a distal end, respectively; an extending direction of the central axis surrounded by the circumference of the stent 1 is parallel to or the same as a distribution direction of the two opposite ends of the stent main body 11.

Specifically, the central axis surrounded by the circumference of the stent 1 extends in an axial direction X.

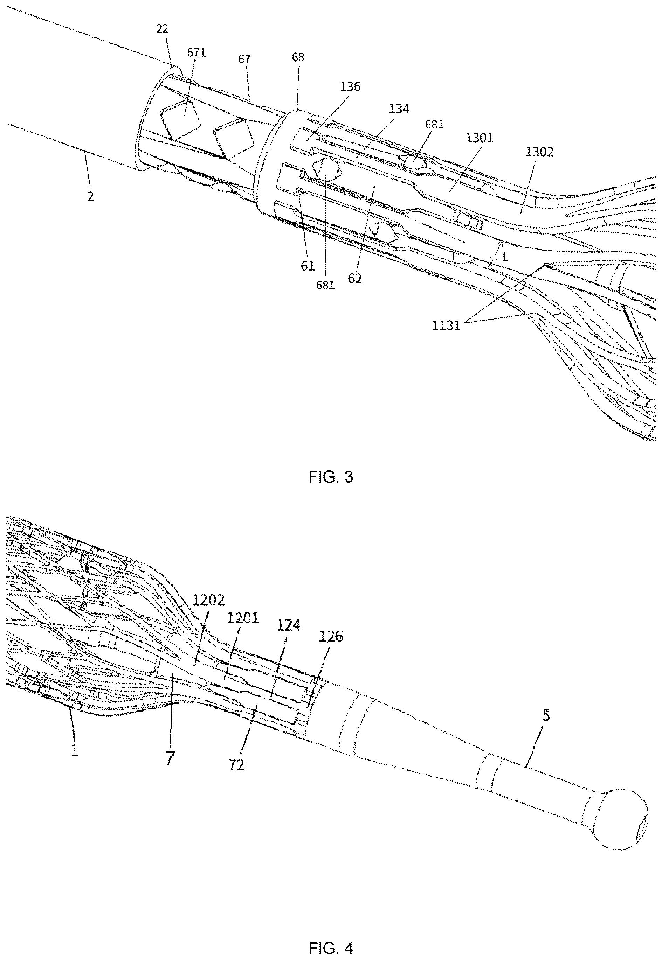

Each of the proximal connecting legs 131 includes a proximal connecting strut 134 and a proximal supporting strut 133 located at a distal end of the proximal connecting strut 134, and the proximal connecting strut 134 and the proximal supporting strut 133 are of an integrated structure. The proximal supporting strut 133 is connected to and supports the stent main body 11, and a circumferential width of the proximal supporting strut 133 is greater than a circumferential width of the proximal connecting strut 134. The proximal connecting strut 134 is fixedly connected to the proximal connecting portion. The proximal supporting strut 133 includes a first connecting portion 1301 fixedly connected to the proximal connecting portion and a first outer side portion 1302 located outside a distal end of the proximal connecting portion. The first connecting portion 1301 and the first outer side portion 1302 are of an integrated structure, with a distal end surface of the proximal connecting portion serving as the boundary therebetween, specifically as indicated by the dashed line in FIG. 3.

It should be noted that two ends of the proximal connecting strut 134 are a proximal end and a distal end, respectively; the proximal connecting portion has two opposite ends, and the two opposite ends of the proximal connecting portion are a proximal end and a distal end, respectively.

Optionally, a distribution direction of the two opposite ends of the proximal connecting portion is parallel to or the same as the axial direction X.

In this embodiment, the circumferential width refers to the dimension of the proximal connecting strut 134 in the circumferential direction of the stent 1, specifically the width L as indicated by the arrow in FIG. 3. The circumferential width of other structures (such as struts, the distal connecting legs 121, and the components included therein) shall be understood in the same manner, and will not be repeated herein.

The plurality of proximal connecting legs 131 are arranged in parallel, and along the circumferential direction of the stent 1, the intervals between adjacent proximal connecting legs 131 are equal, so that the structure of a proximal portion of the stent 1 is more uniform when being stressed, ensuring that the structure of the proximal portion of the stent 1 is more stable, and simplifying the manufacturing process. An extending direction of the proximal connecting legs 131 is parallel to the axial direction X, thereby facilitating connection with the proximal connecting portion (e.g., a first connecting portion 21 or a proximal bearing chamber 6 of the catheter 2). A radial thickness of the proximal connecting leg 131 remains unchanged in the axial direction X. In this way, by varying a circumferential width of the proximal connecting leg 131 at different positions in the axial direction X, the stiffness of the proximal connecting legs 131 at corresponding positions can be adjusted, thereby enabling simple and flexible stiffness adjustment of the proximal connecting legs 131.

It should be noted that the stent 1 has two opposite ends, which are a proximal end and a distal end, respectively; a distribution direction of the two opposite ends of the stent 1 is the same as the distribution direction of the two opposite ends of the stent main body 11.

In addition, the radial thickness of proximal connecting leg 131 is equal to that of the stent main body 11 and that of the distal connecting leg 121. That is, the thickness of a solid structure of the stent 1 at all positions in the axial direction X is the same and uniform. Therefore, the stent 1 can be manufactured by laser cutting from a preformed tube with a uniform wall thickness, resulting in a simple manufacturing process of the stent 1. The solid structure of the stent 1 has the same thickness at all positions, which means that the stent 1 has a uniform wall thickness in the collapsed state, thereby ensuring uniform dimensions of the entire collapsed pump head.

Each of the proximal connecting legs 131 further includes a proximal wide body portion 136 fixedly connected to the proximal connecting portion. The proximal wide body portion 136 is connected to and integrally formed with the proximal connecting strut 134. Preferably, the proximal wide body portion 136 is formed at a proximal end of the proximal connecting strut 134. The proximal connecting strut 134 extends in the axial direction X, and the proximal wide body portion 136 extends in the circumferential direction of the stent 1, the two are arranged substantially perpendicular to each other, and a circumferential width of the proximal wide body portion 136 is greater than the circumferential width of the proximal connecting strut 134, such that the proximal wide body portion 136 and the proximal connecting strut 134 form a substantially "T"-shaped structure. The "T"-shaped structure can be hooked onto a corresponding position of the proximal connecting portion (as described below, snapped in a proximal leg groove 61), achieving a fixed connection of the proximal connecting legs 131 to the proximal connecting portion in both the circumferential direction and the axial direction X of the stent 1. The circumferential width of the proximal connecting strut 134 is smaller than the circumferential width of the proximal supporting strut 133 and also smaller than the circumferential width of the proximal wide body portion 136. Thus, the proximal connecting leg 131 has a circumferential width in the axial direction X that varies rather than being uniform, thereby facilitating quick positioning and assembly with the proximal leg groove 61, and, with the aid of the "T"-shaped structure described above, achieving a fixed connection with the proximal connecting portion in the axial direction X.

In the axial direction X, an axial length of the first connecting portion 1301 is greater than an axial length of the proximal wide body portion 136 but smaller than an axial length of the proximal connecting strut 134. That is, among the portions coinciding with or connected to the proximal connecting portion, the proximal connecting strut 134 is the longest, and the proximal wide body portion 136 is the shortest. By increasing the length of the proximal connecting strut 134 that has a smaller circumferential width (i.e., making the proximal connecting strut 134, which has the smallest circumferential width, the longest), on the one hand, an axial connection length (overlapping length) between the proximal connecting leg 131 and the proximal connecting portion is increased, thereby ensuring the connection strength between the two; on the other hand, a grooving area of the proximal connecting portion can be reduced, ensuring that the structural strength of the proximal connecting portion is not excessively compromised, thereby providing stiffness support for a proximal cantilever structure of the stent 1.

As shown in FIG. 1, the first connecting portion 1301 is linear and extends in the axial direction X, and is disposed together with the proximal connecting strut 134 on an outer wall of a proximal connecting portion. In the radially collapsed state of the stent 1, the first outer side portion 1302 is linear and extends in the axial direction X. In the radially expanded state of the stent 1, the first outer side portion 1302 is curved, and a distance from the first outer side portion 1302 to the central axis of the stent 1 gradually increases from the proximal end to the distal end of the proximal supporting strut 133. The central axis of the stent 1 refers to an axis passing through the center of the stent 1 and extending in the axial direction X. That is, in the radially expanded state, the first outer side portion 1302 extends from the first connecting portion 1301 toward the distal end of the proximal supporting strut 133 in a curved manner to a bifurcation structure 1131, and the bifurcation structure 1131 is a proximal junction of struts forming a mesh in an outlet portion as described below. The first outer side portion 1302 can provide rigidity support for a main body portion of the stent 1 in the radially expanded state.

It should be noted that the two ends of the proximal supporting strut 133 are a proximal end and a distal end, respectively; in the distribution direction of the two ends of the proximal supporting strut 133, the proximal supporting strut 133 includes the first connecting portion 1301 and the first outer side portion 1302 which are connected to each other; an end of the first connecting portion 1301 away from the first outer side portion 1302 serves as the proximal end of the proximal supporting strut 133, and an end of the first outer side portion 1302 away from the first connecting portion 1301 serves as the distal end of the proximal supporting strut 133; and one end of each of the struts is defined as a proximal end thereof.

As shown in FIG. 3, the outer wall of the proximal connecting portion is provided with a plurality of proximal leg grooves 61, in which the plurality of proximal connecting legs 131 are snapped in a one-to-one correspondence, and the shape of the proximal leg groove 61 matches the corresponding shape of the proximal connecting leg 131. A proximal collar 63 is sleeved over the proximal connecting portion and configured to fix the proximal connecting legs 131 in a radial direction of the stent 1 so as to retain the proximal connecting legs 131 within the proximal leg grooves 61. The proximal leg groove 61 is configured to accommodate the proximal wide body portion 136, the proximal connecting strut 134 and the first connecting portion 1301.

When the pump head is collapsed, a sheath applies an axial force in the axial direction X to the expanded stent 1, and the axial force causes the end of the stent 1 to tend to expand radially outward due to the lever principle. However, the tendency of the proximal portion of the stent 1 to expand radially outward will weaken the fixed connection between the proximal end of the stent 1 and the proximal connecting portion, further increasing the possibility of disconnection between the two.

If the stent adopts a secondary connecting tube structure in the prior art, since the secondary connecting tube structure is continuous in the circumferential direction, any portion of the tubular secondary connecting tube in the circumferential direction is interconnected with adjacent portions. This can effectively resist and eliminate the radial outward expansion that occurs when the pump head is collapsed. Therefore, the fixed connection between the stent and the proximal connecting portion can be well maintained.

In contrast, the proximal portion of the stent 1 in this embodiment used for connecting to the proximal connecting portion adopts a discrete leg structure in the circumferential direction of the stent 1. The discrete legs do not generate the interconnected force like that of the secondary connecting tube. Thus, when the pump head is collapsed, the likelihood that the legs will expand radially outward and detach from the proximal connecting portion (specifically, the "T"-shaped structure at the proximal end of the legs will lift out of the leg grooves) is significantly increased, thereby affecting the fixed connection between the stent 1 and the proximal connecting portion.

Therefore, in this embodiment, the proximal collar 63 is sleeved over the proximal connecting portion to resist a radial outward expansion force of the proximal portion of the proximal connecting legs 131, prevent the proximal connecting legs 131 from expanding radially outward and detaching from the proximal connecting portion, ensuring that the "T"-shaped structure formed at the proximal portion of the proximal connecting legs 131 remains in the proximal leg grooves 61 at all times, thereby durably maintaining the fixed connection between the two.

Further, a traditional proximal collar is mostly made of a heat-shrink tubing, mainly for the purpose of simplifying the process. However, the heat-shrink tubing is essentially a plastic tube, which has relatively low strength and is difficult to resist the force of the proximal connecting legs 131 lifting outward. In practice, it has also been found that a proximal collar made of heat-shrink tubing may be punctured by the proximal end of the proximal connecting legs 131. In view of this, the proximal collar 63 in this embodiment is made of a higher-strength metal material (for example, copper or a copper-aluminum alloy), which can avoid the above problems and can more reliably fix and restrain the proximal connecting legs 131 within the proximal leg grooves 61 in the radial direction of the stent 1.

It should be noted that one of the two ends of the proximal connecting leg 131 is a proximal end thereof.

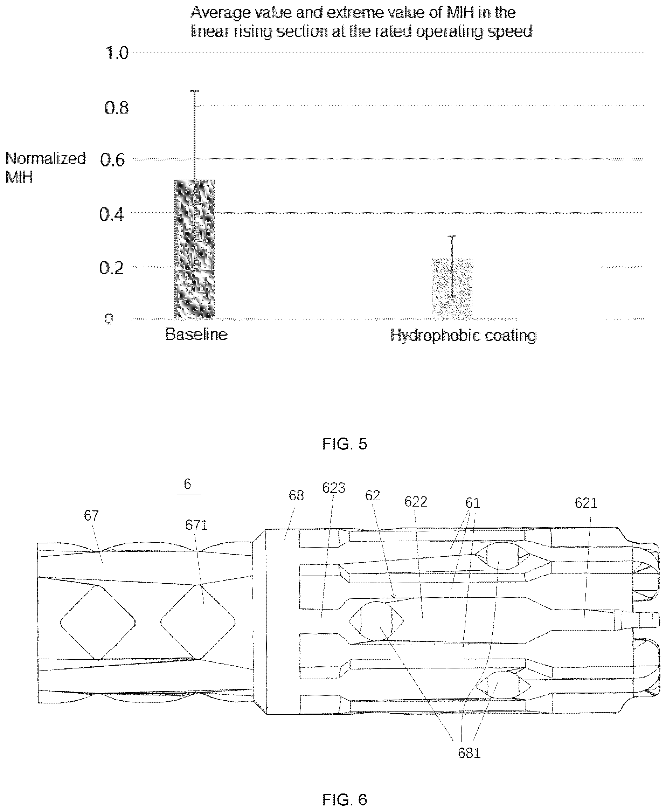

As shown in FIG. 3 and FIG. 6, a proximal spacing protrusion 62 is formed on the outer wall of the proximal connecting portion between two circumferentially adjacent proximal leg grooves 61. A circumferential width of a second protruding portion 622 of the proximal spacing protrusion 62 between the proximal connecting struts 134 is greater than the circumferential width of the proximal connecting strut 134. A circumferential width of a third protruding portion 623 of the proximal spacing protrusion 62 between the proximal wide body portions 136 is smaller than the circumferential width of the proximal wide body portion 136, and a circumferential width of a first protruding portion 621 of the proximal spacing protrusion 62 between the first connecting portions 1301 is smaller than a circumferential width of the first connecting portion 1301.

Specifically, the proximal spacing protrusion 62 includes the first protruding portion 621 located between two adjacent first connecting portions 1301, the second protruding portion 622 located between two adjacent proximal connecting struts 134, and the third protruding portion 623 located between two adjacent proximal wide body portions 136. Essentially, the circumferential width of the second protruding portion 622 is greater than that of the first protruding portion 621 and greater than that of the third protruding portion 623. Since the "T"-shaped structure formed by the proximal wide body portion 136 and the proximal connecting strut 134 forms a hook-type connection with the proximal end of the second protruding portion 622 of the proximal spacing protrusion 62, the circumferential width of the second protruding portion 622 is relatively large, so that the second protruding portion 622 has a relatively high strength, thereby providing a high-strength limiting effect for the proximal "T"-shaped structure of the stent 1. This prevents a problem where a material collapse occurs at the proximal end of the second protruding portion 622 of the proximal spacing protrusion 62 when the stent 1 exerts an axial force toward the distal end on the proximal connecting portion during collapsing, thereby ensuring that the above hook-type connection can be stably maintained.

It should be noted that the second protruding portion 622 has two opposite ends, one of the two opposite ends of the second protruding portion 622 is a proximal end; when the "T"-shaped structure formed by the proximal wide body portion 136 and the proximal connecting strut 134 forms a hook-type connection with the proximal end of the second protruding portion 622 of the proximal spacing protrusion 62, a distribution direction of the two opposite ends of the second protruding portion 622 is parallel or the same as the extending direction of the proximal connecting strut 134.

The circumferential width of the proximal connecting strut 134 is uniform, the circumferential width of the proximal supporting strut 133 is also uniform, and the circumferential width of the proximal wide body portion 136 is also uniform. That is, the circumferential width of the proximal connecting strut remains unchanged in the extending direction thereof, and the circumferential width of the proximal connecting strut 134 remains unchanged in the axial direction X; the circumferential width of the proximal supporting strut remains unchanged in the extending direction thereof, and the circumferential width of the proximal supporting strut 133 remains unchanged in the axial direction X; the circumferential width of the proximal wide body portion remains unchanged in the extending direction of the proximal connecting strut, and the circumferential width of the proximal wide body portion 136 remains unchanged in the axial direction X. In this way, the structure of the proximal connecting leg 131 can be simplified as much as possible, and more importantly, the manufacturing process of the proximal connecting leg 131 and of the proximal leg groove 61 that cooperates therewith can be simplified.

The reason for achieving the above effects is as follows: the proximal connecting leg 131 already includes a plurality of portions with different widths (i.e., the proximal connecting strut 134, the proximal supporting strut 133, and the proximal wide body portion 136). If the proximal connecting strut 134, and/or the proximal supporting strut 133, and/or the proximal wide body portion 136 are designed to have a varying circumferential width, for example, if the proximal connecting strut 134 and/or the proximal supporting strut 133 are designed to be gradually wider or narrower from the respective proximal ends to distal ends, this can significantly increase the complexity of the manufacturing process for the proximal connecting leg 131. Moreover, such a complex structural design of the proximal connecting leg 131 would not significantly improve performance in other aspects (e.g., the connection strength with the proximal connecting portion). On the contrary, it can cause the width of the matching proximal leg groove 61 to change accordingly, thereby complicating the formation of the proximal leg groove 61. However, the uniform axial width of the proximal connecting strut 134, the proximal supporting strut 133, and the proximal wide body portion 136 can avoid the above-mentioned problems.

A proximal transition portion 135 is provided between the proximal supporting strut 133 and the proximal connecting strut 134, and in a direction from the proximal end to a distal end of the proximal transition portion 135, a circumferential width of the proximal transition portion 135 gradually increases from being equal to that of the proximal connecting strut 134 to being equal to that of the proximal supporting strut 133. The aforesaid gradual increase may be a linear increase or an exponential increase, which is structurally reflected in the proximal transition portion 135 as a beveled transition or a curved transition, respectively. Providing the proximal transition portion 135 allows the connected proximal supporting strut 133 and proximal connecting strut 134 to exhibit a gradual change in circumferential width rather than an abrupt change. This prevents stress concentration at the connection between the proximal supporting strut 133 and the proximal connecting strut 134 caused by an abrupt change in circumferential width, thereby avoiding fracture at the connection between the two.

It should be noted that the proximal transition portion 135 has two opposite ends, which are a proximal end and a distal end, respectively; the proximal end of the proximal transition portion 135 is connected to the proximal connecting strut 134, and the distal end of the proximal transition portion 135 is connected to the proximal supporting strut 133.

As shown in FIG. 3, in one embodiment, the proximal connecting portion is a proximal bearing chamber 6 provided at the distal end of the catheter 2. A proximal bearing is disposed inside the proximal bearing chamber 6, and a drive shaft (specifically a rigid shaft described below) passes through the proximal bearing. The drive shaft is connected to the impeller and is used to drive the impeller to rotate. The proximal leg grooves 61 are provided on an outer wall surface at the distal end of the proximal bearing chamber 6, and the proximal collar 63 is sleeved on the outer wall surface of the proximal bearing chamber 6 where the proximal leg grooves 61 are provided.

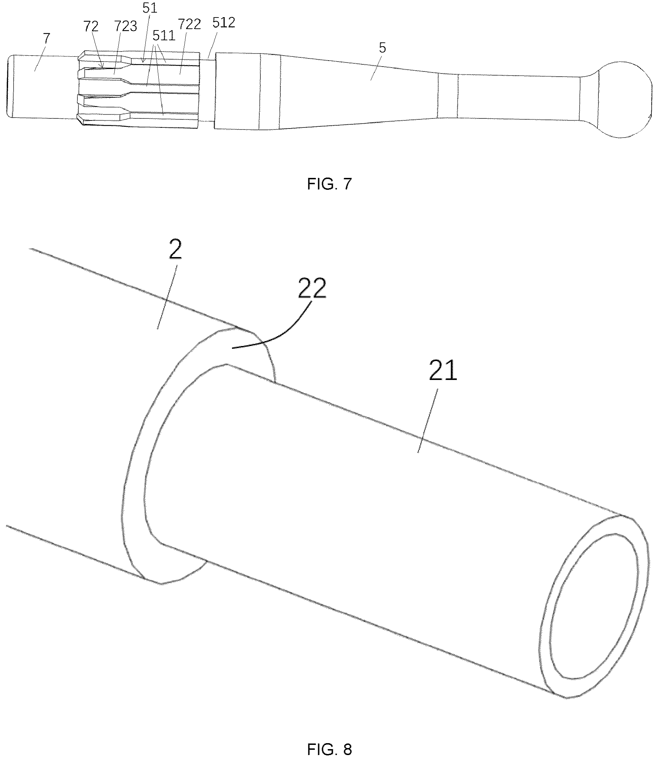

As shown in FIG. 8, an outer wall of the distal end of the catheter 2 is reduced in diameter to form the first connecting portion 21; specifically, the thickness of the outer wall surface of a distal section of the catheter 2 is reduced to form the first connecting portion 21. A stop step 22 is formed between the first connecting portion 21 and the adjacent distal portion of the catheter 2, which provides a limit stop for the proximal bearing chamber 6 and facilitates positioning and installation of the two.

As shown in FIG. 6, two axial ends of the proximal bearing chamber 6 are a proximal end and a distal end, respectively; and the proximal bearing chamber 6 is of a stepped structure, including a first portion 67 located at the proximal end of the proximal bearing chamber 6 and a second portion 68 located at the distal end of the proximal bearing chamber 6. The inner diameter of the first portion 67 is equal to that of the second portion 68, but the outer diameter of the first portion 67 is smaller than that of the second portion 68. The outer diameter of the second portion 68 is smaller than the outer diameter of the catheter 2, the first portion 67 is sleeved over the first connecting portion 21, and the proximal leg grooves 61 are provided on an outer wall surface of the second portion 68. The first portion 67 is provided with through holes 671 that extend through inner and outer surfaces thereof in a radial direction of the proximal bearing chamber 6. When assembling the proximal bearing chamber 6 and the catheter 2, first, a heat-shrink tubing 20 is sleeved over the first portion 67 of the proximal bearing chamber 6, and then the first portion 67 of the proximal bearing chamber 6 is sleeved over the first connecting portion 21 of the catheter 2. Subsequently, a heat-shrink process is performed on the heat-shrink tubing 20; the material of the heat-shrink tubing 20 melts and flows into the through holes 671 and adheres to an outer wall of the first connecting portion 21 of the catheter 2. Meanwhile, the inner wall material of the heat-shrink tubing 20, after being heat-shrunk, also adheres to an outer wall of the first portion 67, thereby securing the proximal bearing chamber 6 to the catheter 2. Preferably, the material of the heat-shrink tubing 20 is the same as that of the catheter 2, such that the heat-shrink tubing 20 and the catheter 2 have the same molecular structure, thereby enhancing the bonding strength between the melted heat-shrink tubing 20 material and the catheter 2 material, and thus improving the effect of the fixed connection between the proximal bearing chamber 6 on the catheter 2.

Since the outer diameter of the first portion 67 is relatively small, and the first connecting portion 21 is formed by reducing the thickness of the outer wall of the distal end of the catheter 2, the outer wall of the first portion 67 is not flush with the outer wall of the distal end of the catheter 2 after the first portion 67 of the proximal bearing chamber 6 is sleeved over the first connecting portion 21, specifically, the outer wall of the first portion 67 is lower than the outer wall of the distal end of the catheter 2.

After heat shrinking, the outer diameter of the heat-shrink tubing 20 is substantially equal to that of the catheter 2. Therefore, sleeving the heat-shrink tubing 20 over the first portion 67 not only achieves the fixed connection between the proximal bearing chamber 6 and the catheter 2, but also fills a height difference between the outer wall of the first portion 67 and the outer wall of the distal end of the catheter 2, so that the outer wall of the catheter 2 is flush with the outer wall of the heat-shrink tubing 20, thereby avoiding unevenness in height that could otherwise cause hemolysis and thrombosis.

The outer wall surface of the second portion 68 is provided with a plurality of recesses 681, and each recess 681 is located between two adjacent proximal leg grooves 61. Specifically, the recesses 681 are formed on the second protruding portion 622 of the proximal spacing protrusion 62. After the proximal connecting legs 131 of the stent 1 are snapped into the proximal leg grooves 61, the proximal collar 63 is sleeved over, and then the proximal collar 63 is pressed downward at the position corresponding to the recesses 681. The proximal collar 63 deforms inward and is snapped in the recesses 681, thereby achieving the fixation of the proximal collar 63 on the outer wall of the proximal bearing chamber 6. Since the outer diameter of the second portion 68 is smaller than that of the catheter 2, as shown in FIG. 2, similarly, the outer diameter of the proximal collar 63 is approximately the same as the outer diameter of the catheter 2 and the outer diameter of the heat-shrink tubing 20 after heat shrinking, which allows the proximal collar 63 to radially wrap and fix the proximal connecting legs 131 while being flush with the outer walls of the catheter 2 and the heat-shrink tubing 20, thus preventing the problems of hemolysis and thrombosis.

Alternatively, in another embodiment, the proximal connecting portion may also include the aforementioned first connecting portion 21 formed at the distal end of the catheter 2. Similarly, in this embodiment, the thickness of the outer wall at the distal end of the catheter 2 is reduced to form the first connecting portion 21 (i.e., the proximal connecting portion). The main difference between this embodiment and the previous one lies in the structure of the proximal connecting portion; most other structures are the same. For example, the proximal leg grooves 61 are formed on the outer wall of the first connecting portion 21, the proximal connecting legs 131 are snapped in the proximal leg grooves 61, and then the proximal collar 63 is sleeved over the first connecting portion 21 to radially fix the proximal connecting legs 131, and restraining and retaining the proximal connecting legs in the proximal leg grooves 61.

Moreover, since in this embodiment the proximal bearing chamber 6 is omitted from connecting the catheter 2 and the stent 1, and the catheter 2 is directly connected to the stent 1, another difference between this embodiment and the previous embodiment is that only a proximal collar 63 is disposed in this embodiment, and no heat-shrink tubing 20 is required, making the structure relatively simpler.

As described above, compared with the previous embodiment, the proximal bearing chamber 6 is omitted in this embodiment; therefore, the proximal bearing for supporting the proximal end of the drive shaft needs to be alternatively provided by other structures. For example, the proximal bearing may be disposed inside the proximal end of the stent 1, i.e., clamped and fixed by the plurality of proximal connecting legs 131.

It should be noted that the two ends of the drive shaft are referred to as the proximal end and the distal end, respectively.

Further referring to FIG. 1, the stent 1 further includes distal connecting legs 121 located at the distal end of the stent main body 11. Each of the distal connecting legs 121 has the same structure as the proximal connecting legs 131. Therefore, regarding the specific structure of the distal connecting legs 121 and the description of the corresponding effects thereof, reference may be made to the above description of the proximal connecting legs 131; and the identical parts will not be redundantly described. The following mainly focuses on the parts of the distal connecting legs 121 that differ from the proximal connecting legs 131.

It should be noted that two ends of the distal connecting leg 121 are referred to as a proximal end and a distal end, respectively.

A plurality of distal connecting legs 121 are provided, which are arranged at intervals along a circumferential direction of the stent 1, and each distal connecting leg 121 includes a distal connecting strut 124 and a distal supporting strut 123 located at a proximal end of the distal connecting strut 124. The distal supporting strut 123 is connected to and supports the stent main body 11. A circumferential width of the distal supporting strut 123 is greater than that of the distal connecting strut 124. The distal supporting strut 123 includes a second connecting portion 1201 fixedly connected to the distal connecting portion and a second outer side portion 1202 located outside a proximal end of the distal connecting portion.

It should be noted that two ends of the distal connecting strut 124 are a proximal end and a distal end, respectively; the distal connecting portion has two opposite ends, and the two opposite ends of the distal connecting portion are a proximal end and a distal end, respectively.

Optionally, a distribution direction of the two opposite ends of the distal connecting portion is parallel to or the same as the axial direction X.

The plurality of distal connecting legs 121 are arranged in parallel, and in the circumferential direction of the stent 1, the intervals between adjacent distal connecting legs 121 are equal, so that the structure of a distal portion of the stent 1 is more uniform when being stressed, ensuring that the structure of the distal portion of the stent 1 is more stable, and simplifying the manufacturing process. An extending direction of the distal connecting legs 121 is parallel to the axial direction X, thereby facilitating connection with the distal connecting portion (e.g., a distal bearing chamber 7 and/or a protective tip 5). A radial thickness of the distal connecting leg 121 remains unchanged in the axial direction X. In this way, by varying a circumferential width of the distal connecting leg 121 at different positions in the axial direction X, the stiffness of the distal connecting legs 121 at corresponding positions can be adjusted, thereby enabling simple and flexible stiffness adjustment of the distal connecting legs 121.

Each of the distal connecting legs 121 further includes a distal wide body portion 126 fixedly connected to the distal connecting portion, the distal wide body portion 126 is connected to and integrally formed with the distal connecting strut 124, and preferably, the distal wide body portion 126 is formed at a distal end of the distal connecting strut 124. The distal wide body portion 126 and the distal connecting strut 124 form a substantially "T"-shaped structure. The "T"-shaped structure can be hooked onto a corresponding position of the distal connecting portion (as described below, snapped in a distal leg groove 51), achieving a fixed connection of the distal connecting legs 121 to the distal connecting portion in both the circumferential direction and the axial direction X of the stent 1.

A circumferential width of the distal connecting strut 124 is smaller than the circumferential width of the distal supporting strut 123 and also smaller than a circumferential width of the distal wide body portion 126. Thus, the distal connecting leg 121 has a circumferential width in the axial direction X that varies rather than being uniform, thereby facilitating quick positioning and assembly with the distal leg groove, and, with the aid of the "T"-shaped structure described above, achieving a fixed connection with the distal connecting portion in the axial direction X.

In the axial direction X, an axial length of the second connecting portion 1201 is greater than an axial length of the distal wide body portion 126 but smaller than an axial length of the distal connecting strut 124. Thus, on the one hand, an axial connection length (overlap length) between the distal connecting legs 121 and the distal connecting portion is increased to ensure the connection strength between the two. On the other hand, a grooving area of the distal connecting portion can be reduced, ensuring that the structural strength of the distal connecting portion is not excessively compromised. This, in turn, provides good structural support for a distal cantilever structure of the stent 1.

An outer wall of the distal connecting portion is provided with a plurality of distal leg grooves 51, in which the plurality of distal connecting legs 121 are snapped in a one-to-one correspondence. Each distal leg groove 51 includes an axial groove portion 511 extending substantially in the axial direction X and a distal circumferential annular groove 512 located distally of and in communication with the axial groove portion 511. The distal connecting strut 124 and the second connecting portion 1201 of the distal connecting leg 121 are snapped in the axial groove portion 511, and the distal wide body portion 126 is snapped in the distal circumferential annular groove 512.

Accordingly, the difference between the distal leg groove 51 and the proximal leg groove 61 lies in that a groove body portion for accommodating the distal wide body portion 126 is continuous in the circumferential direction of the stent 1, thereby forming the distal circumferential annular groove 512. A groove body for accommodating the proximal wide body portion 136 forms a portion of the structure of the proximal leg groove 61, and groove bodies are not in communication with each other in the circumferential direction of the stent 1 to constitute a circumferentially continuous annular groove structure.

However, it should be noted that the two structures can be referenced and replaced with each other. That is, a portion for accommodating the distal wide body portion 126 may form a portion of the structure of the distal leg groove 51, and it is not necessary to provide the circumferentially continuous annular groove structure. Similarly, the groove body portion for accommodating the proximal wide body portion 136 may be continuous in the circumferential direction of the stent 1 to form a proximal circumferential annular groove.

Similarly, a distal collar 73 is sleeved over the distal connecting portion and configured to fix the distal connecting legs 121 in the radial direction of the stent 1 so as to retain the distal connecting legs 121 within the distal leg grooves 51. The distal collar 73 also functions to prevent the distal connecting legs 121 from lifting when the stent 1 is collapsed, and is made of a metal material with higher strength.

As shown in FIG. 4 and FIG. 7, a distal spacing protrusion 72 is formed on the outer wall of the distal connecting portion between two circumferentially adjacent distal leg grooves 51 (specifically, the axial groove portion 511). The distal spacing protrusion 72 includes a fourth protruding portion 723 located between two adjacent second connecting portions 1201 and a fifth protruding portion 722 located between two adjacent distal connecting struts 124. As described above, when a portion for accommodating the distal wide body portion 126 is a portion of the structure of the distal leg groove 51, and no continuous annular groove structure is present in the circumferential direction of the stent 1. The distal spacing protrusion 72 further includes a sixth protruding portion located between two adjacent distal wide body portions 126 (the accompanying drawings are illustrated with the presence of the distal circumferential annular groove 512, so such sixth protruding portion is not present herein).

Compared with the fourth protruding portion 723 and the sixth protruding portion, the fifth protruding portion 722 has the largest circumferential width. Therefore, the fifth protruding portion 722 has a relatively higher strength, thereby providing a high-strength limiting effect for the "T"-shaped structure at the distal end of the stent 1 and ensuring the stability of a hook-type connection formed between the distal connecting legs 121 and the distal connecting portion.

The circumferential width of each of the distal connecting strut 124, the distal supporting strut 123, and the distal wide body portion 126 is uniform, which simplifies the structure of the distal connecting legs 121 and the manufacturing process of the distal connecting legs 121 and the distal leg grooves 51. Specifically, the circumferential width of the distal connecting strut 124 remains unchanged in an extending direction thereof, the circumferential width of the distal supporting strut 123 remains unchanged in an extending direction thereof, and the circumferential width of the distal wide body portion 126 remains unchanged in an extending direction of the distal connecting strut 124.

A distal transition portion 125 is provided between the distal supporting strut 123 and the distal connecting strut 124. In a direction from a proximal end to a distal end of the distal transition portion 125, a circumferential width of the distal transition portion 125 gradually increases from being equal to that of the distal supporting strut 123 to being equal to that of the distal connecting strut 124, so as to avoid stress concentration, thereby avoiding fracture at the connection between the two.

It should be noted that the distal transition portion 125 has two opposite ends, which are a proximal end and a distal end, respectively; the proximal end of the distal transition portion 125 is connected to the distal connecting strut 123, and the distal end of the distal transition portion 125 is connected to the distal supporting strut 124.

In one embodiment, the distal connecting portion is a distal bearing chamber 7 connected to the distal end of the stent 1. A distal bearing is provided in the distal bearing chamber 7, and the distal end of the drive shaft (specifically, a rigid shaft described below) passes through the distal bearing. A distal end of the distal bearing chamber 7 is connected to the protective tip 5. The connection between the distal bearing chamber 7 and the distal connecting legs 121 may refer to the connection scheme between the proximal bearing chamber 6 and the proximal connecting legs 131. The connection between the protective tip 5 and the distal bearing chamber 7 may be such that a proximal end of the protective tip 5 is inserted into the distal bearing chamber 7. Specifically, reference can be made to a known embodiment disclosed in Publication No. CN216908915U, the identical parts of which are incorporated herein by reference and will not be repeated herein. At this point, the protective tip 5 is relatively thin and may adopt a traditional "J"-shaped pigtail structure.

It should be noted that two axial ends of the distal bearing chamber 7 are a proximal end and a distal end, respectively.

In another embodiment, the distal connecting portion may also include the protective tip 5. In this embodiment, the protective tip 5 is relatively thick and generally has a straight-tip structure, which is sleeved over an outer side of the distal end of the distal bearing chamber 7. Regarding the structure and connection relationship between the protective tip 5 and the distal bearing chamber 7 in this embodiment, and other related structural features (e.g., a hemostatic valve, etc.), reference may be made to the known embodiment disclosed in Publication No. CN115154892A, the identical parts of which are incorporated herein by reference, and will not be repeated here.

Of course, in the embodiment adopting the protective tip 5 with the straight-tip structure, the distal bearing chamber 7 is not an essential structure and may be omitted in the same manner as the proximal bearing chamber 6. In this case, similarly to the above, the distal bearing may be disposed within the distal end of the stent 1, i.e., clamped and fixed by the plurality of distal connecting legs 121.

As shown in FIG. 1, the distal connecting legs 121 and the proximal connecting legs 131 have identical structures and are arranged symmetrically with respect to a central plane of the stent main body 11. The central plane passes through the center of the stent main body 11 and is perpendicular to the axial direction X.

As shown in FIG. 1, a plurality of mesh holes 114 are distributed on the stent main body 11, and each mesh hole 114 is defined by at least two pairs of parallel linear struts 115. Specifically, each mesh hole 114 includes two parallel first struts 116 and two parallel second struts 117. Both the first struts 116 and the second struts 117 are linear as a whole, and the first struts 116 and the second struts 117 have the same length. A plurality of struts 115 of each mesh hole 114 enclose a polygonal mesh hole. Each strut 115 is linear as a whole and may be of a non-bent linear type. Alternatively, the struts 115 may also be straight struts that allow slight bending but can still be intuitively regarded as straight struts of a polygon. In the embodiments of the present disclosure, the strut 115 may have a linear structure as a whole.

Referring to Fig. 2, the catheter pump according to the embodiment of the present disclosure includes a power assembly (not shown) and a working assembly. The power assembly includes a housing and a motor accommodated in the housing and having an output shaft. The working assembly includes a catheter 2, a drive shaft disposed within the catheter 2, and a pump head. The pump head can be delivered through the catheter 2 to a desired position in the heart, for example, to pump blood into the left ventricle. The pump head includes a pump housing having a blood inlet and a blood outlet, and an impeller accommodated in the pump housing. The blood inlet is located at, and the blood outlet is located at a proximal end of the pump housing. The motor is disposed at a proximal end of the catheter 2 and connected to the catheter 2 via a coupler, and drives the impeller to rotate for blood pumping via the drive shaft.

It should be noted that the pump housing has two opposite ends, which are a proximal end and a distal end, respectively.

Optionally, a distribution direction of the two opposite ends of the pump housing is the same as the distribution direction of the two opposite ends of the stent 1.

The pump housing is connected to the distal end of the catheter 2, and the impeller is connected to a distal end of the drive shaft. The pump housing includes a membrane (not shown) defining a blood flow channel, and a collapsible stent 1 that supports and expands the membrane. The proximal end of the stent 1 is connected to the distal end of the catheter 2. The stent 1 is any of the stents 1 described in the foregoing embodiments, and the proximal connecting legs 131 of the stent 1 are connected to the distal end of the catheter 2.

The membrane covers the outer side of a portion of the stent 1; a portion of the stent 1 is disposed inside the membrane, and another portion is disposed outside the membrane. The impeller is accommodated inside the stent 1 and located within the membrane. The stent 1 is supported at a distal end of the membrane; a portion of the stent 1 is located inside the membrane, and another portion is located outside the distal end of the membrane. Most of the impeller is located inside the main body portion of the stent 1, and the two ends (mainly the hub) extend into an inlet portion and an outlet portion of the stent 1.

It should be noted that the membrane has two opposite ends, one of which is defined as a distal end thereof. A distribution direction of the two opposite ends of the membrane is the same as that of the two opposite ends of the stent 1.

The membrane has a cylindrical segment serving as a main structure, and a conical segment located at a proximal end of the cylindrical segment; one of two ends of the cylindrical segment is a proximal end thereof, and one of two ends of the conical segment is a proximal end thereof. The proximal end of the conical segment is disposed outside the catheter 2 and fixed to the outer wall of the catheter 2. The catheter 2 is connected to the proximal end of the stent 1 via the proximal bearing chamber 6 located at the distal end thereof, and a proximal bearing for rotatably supporting the drive shaft is disposed inside the proximal bearing chamber 6.

A distal bearing chamber 7 is provided at the distal end of the stent 1, and a distal bearing for rotatably supporting the distal end of the drive shaft is disposed inside the distal bearing chamber 7. The drive shaft includes a flexible shaft passing through the catheter 2, and a rigid shaft connected to a distal end of the flexible shaft. A hub of the impeller is sleeved over the rigid shaft, and a proximal end and a distal end of the rigid shaft pass through the proximal bearing and the distal bearing, respectively. By means of the rigid shaft and the bearings at the two ends, stiffness support is provided for the impeller in the pump housing to maintain the stable position of the impeller in the pump housing.

It should be noted that one of the two ends of the flexible shaft is a distal end thereof, and two ends of the rigid shaft are a proximal end and a distal end, respectively.

The two axial ends of the proximal bearing are a proximal end and a distal end, respectively. Two axial sides of the impeller are a proximal side and a distal side thereof, respectively. A stopper located on the proximal side of the proximal bearing is disposed on the rigid shaft and is configured to limit the movement of the rigid shaft and the impeller toward the distal side so as to prevent the impeller from moving distally during rotational blood pumping caused by the reverse action of blood. A limiting member located on the proximal side of the stopper is further disposed on the rigid shaft, which is configured to restrict the movement of the rigid shaft and the stopper toward the proximal side of the rigid shaft, thereby avoiding the stopper from causing eccentric wear on the distal end of catheter 2 and resulting in the release of particles.

A coupler is connected to the proximal end of the catheter 2. A liquid flow channel is provided between the catheter 2 and the drive shaft, and a flushing fluid flowing in the liquid flow channel can provide lubrication and cooling for the rotation of the drive shaft. The coupler is provided with a flushing fluid inlet, which is in communication with the liquid flow channel.

The distal end of the distal bearing chamber 7 is provided with a flexible protective tip 5. The protective tip 5 is supported on a ventricular wall in a non-invasive or atraumatic manner to separate the blood inlet of the pump head from the ventricular wall, thereby preventing a suction inlet of the pump head from adhering to the ventricular wall due to a reaction force of blood during operation and ensuring an effective suction area of the pump.

The pump housing includes a radially collapsed state adapted for intervention in or delivery through a vascular system of a subject, and a natural expanded state corresponding to when the impeller is not rotating. By providing the collapsible pump housing, the pump housing has a small collapsed size and a large expanded size, thereby meeting the dual requirements of reducing the pain of the subject and facilitating intervention during the intervention and delivery process, and ensuring a high blood flow.

The pump head has an intervention configuration and an operational configuration. When the pump head is in the intervention configuration, the pump housing and the impeller are in a radially collapsed state, enabling the pump head to be introduced into the vascular system of the subject or delivered within the vascular system with a small size. When the pump head is in the operational configuration, the pump housing and the impeller are in a radially expanded state, enabling the pump head to pump blood in the left ventricle with a large size.

The radially expanded state of the pump housing includes the above natural expanded state and the operational expanded state when the impeller rotates, and the natural expanded state and the operational expanded state are different states before and after impeller rotation. The stent 1 has a straight-tube structure in the radially collapsed state and has a spindle structure in the radially expanded state. The axial length of the stent 1 in the radially collapsed state is greater than that in the radially expanded state.

The design of the polygonal mesh holes (especially the rhombic mesh holes) of the stent 1 enables optimal collapse, while the shape memory property of a nickel-titanium alloy facilitates the expansion. Corresponding to the intervention configuration and the operational configuration of the pump head, the stent 1 is operable to switch between the radially collapsed state and the radially expanded state.

In the radially expanded state, the stent main body 11 includes a substantially cylindrical main body portion and substantially conical portions disposed at both ends of the main body portion in the axial direction X. The conical portion disposed at the distal end of the main body portion serves as the inlet portion. A distal end of the inlet portion is connected to the distal connecting legs 121, and is connected to the distal bearing chamber 7 or the protective tip 5 via the distal connecting legs 121. The conical portion disposed at a proximal end of the main body portion serves as the outlet portion. A proximal end of the outlet portion is connected to proximal connecting legs 131, and is connected to the proximal bearing chamber 6 or the catheter 2 via the proximal connecting legs 131.

It should be noted that, in the axial direction of the main body portion, two ends of the main body portion are a distal end and a proximal end, respectively. One end of the inlet portion is defined as the distal end; and one end of the outlet portion is defined as the proximal end.

The stent 1 is manufactured by a laser cutting process, and subsequently subjected to posttreatment after the laser cutting to remove burrs and improve surface roughness. During rotation, the impeller drives blood to collide and cut against a surface of the stent 1, resulting in damage to blood cells and severe hemolysis. To solve this problem, in one embodiment, a hydrophobic coating is disposed on at least a surface of the main body portion of the stent 1 to improve hemolysis conditions. Preferably, a hydrophobic coating is provided on a surface of the stent main body 11. More preferably, a hydrophobic coating is provided on the entire surface of the stent 1. The hydrophobic coating can significantly reduce a surface friction coefficient of the stent 1, minimize the frictional shear between blood and the inner wall of the stent when blood flows inside the stent, thereby reducing the damage to blood cells and avoiding severe hemolysis. Specifically, the hydrophobic coating in this embodiment may adopt a PTFE hydrophobic coating.

As shown in FIG. 5, under the condition that other factors are the same, normalized mechanical intravascular hemolysis (MIH) values of the stent without the hydrophobic coating (baseline) and the stent with the hydrophobic coating (optimized scheme) are shown when the impeller performs rotary blood pumping at a rated operating speed. The columns represent the average values of the normalized MIH, and the lines represent the (upper and lower) extreme values of the normalized MIH. The extreme value range of the normalized MIH of a baseline stent 20511 is approximately between 0.19 and 0.88, with an average value of approximately 0.54. In contrast, the extreme value range of the MIH of an optimized stent is approximately between 0.1 and 0.3, with an average value of approximately 0.2.

It can be seen that after the stent is coated with a hydrophobic coating, the MIH value characterizing the hemolysis index decreases significantly. Specifically, the lower extreme value decreases by 49%, the upper extreme value decreases by 65%, and the average value decreases by 62%.

The technical features of the above embodiments may be combined arbitrarily. For the sake of concise description, not all possible combinations of the technical features in the above embodiments have been described; however, as long as there is no contradiction in the combination of these technical features, all such combinations shall be deemed to fall within the scope recorded in this specification.

The above embodiments merely represent several implementations of the present disclosure. The descriptions are relatively specific and detailed, but should not be construed as limiting the scope of the present disclosure. It is to be noted that several variations and modifications may also be made by those of ordinary skill in the art without departing from the conception of the present disclosure, which all fall within the scope of protection of the present disclosure. Therefore, the scope of protection of the present disclosure shall be subject to the appended claims.

## Claims

1. A catheter pump, comprising:
a catheter;
a proximal connecting portion, connected to a distal end of the catheter; and
a pump head, comprising a stent and an impeller accommodated in the stent, wherein the impeller is configured to be driven to rotate for blood pumping,
wherein the stent is operable to switch between a radially collapsed state and a radially expanded state; in the radially expanded state, the stent comprises a stent main body and a plurality of proximal connecting legs located at a proximal end of the stent main body, and the plurality of proximal connecting legs are arranged at intervals along a circumferential direction of the stent;
each of the proximal connecting legs comprises a proximal connecting strut and a proximal supporting strut located at a distal end of the proximal connecting strut; the proximal supporting strut is connected to and supports the stent main body, and a circumferential width of the proximal supporting strut is greater than a circumferential width of the proximal connecting strut; and
the proximal connecting strut is fixedly connected to the proximal connecting portion; and the proximal supporting strut comprises a first connecting portion fixedly connected to the proximal connecting portion and a first outer side portion located outside a distal end of the proximal connecting portion.

2. The catheter pump as claimed in claim 1, wherein each of the proximal connecting legs further comprises a proximal wide body portion fixedly connected to the proximal connecting portion, and a circumferential width of the proximal wide body portion is greater than a circumferential width of the proximal connecting strut; and the proximal wide body portion is located at a proximal end of the proximal connecting strut.

3. The catheter pump as claimed in claim 2, wherein an axial length of the first connecting portion is greater than an axial length of the proximal wide body portion and less than an axial length of the proximal connecting strut.

4. The catheter pump as claimed in claim 2, wherein an outer wall of the proximal connecting portion is provided with a plurality of proximal leg grooves, in which the plurality of proximal connecting legs are snapped in a one-to-one correspondence, and a proximal collar is sleeved over the proximal connecting portion and configured to fix the proximal connecting legs in a radial direction of the stent so as to retain the proximal connecting legs within the proximal leg grooves.

5. The catheter pump as claimed in claim 4, wherein
a proximal spacing protrusion is formed on the outer wall of the proximal connecting portion between two circumferentially adjacent proximal leg grooves, the proximal spacing protrusion comprises a first protruding portion located between two adjacent first connecting portions and a second protruding portion located between two adjacent proximal connecting struts, and a circumferential width of the second protruding portion is greater than a circumferential width of the first protruding portion; and/or
the proximal collar is made of a metallic material.

6. The catheter pump as claimed in claim 2, wherein
the circumferential width of the proximal connecting strut remains unchanged in an extending direction thereof; and/or
the circumferential width of the proximal supporting strut remains unchanged in an extending direction thereof; and/or
the circumferential width of the proximal wide body portion remains unchanged in the extending direction of the proximal connecting strut.

7. The catheter pump as claimed in claim 1, wherein a proximal transition portion is provided between the proximal supporting strut and the proximal connecting strut, and in a direction from a proximal end of the proximal transition portion to a distal end of the proximal transition portion, a circumferential width of the proximal transition portion gradually increases from being equal to that of the proximal connecting strut to being equal to that of the proximal supporting strut.

8. The catheter pump as claimed in claim 1, wherein the proximal connecting portion is a proximal bearing chamber provided at the distal end of the catheter, a proximal bearing is disposed in the proximal bearing chamber, and a drive shaft passes through the proximal bearing and is connected to the impeller so as to drive the impeller to rotate.

9. The catheter pump as claimed in claim 1, wherein the stent further comprises a plurality of distal connecting legs located at a distal end of the stent main body, and the plurality of proximal connecting legs are arranged at intervals along a circumferential direction of the stent;
each of the distal connecting legs comprises a distal connecting strut and a distal supporting strut located at a proximal end of the distal connecting strut; the distal supporting strut is connected to and supports the stent main body, and a circumferential width of the distal supporting strut is greater than a circumferential width of the distal connecting strut; and
the distal connecting strut is fixedly connected to a distal connecting portion; and the distal supporting strut comprises a second connecting portion fixedly connected to the distal connecting portion and a second outer side portion located outside a proximal end of the distal connecting portion.

10. The catheter pump as claimed in claim 9, wherein each of the distal connecting legs further comprises a distal wide body portion fixedly connected to the distal connecting portion, and a circumferential width of the distal wide body portion is greater than a circumferential width of the distal connecting strut; and the distal wide body portion is located at a distal end of the distal connecting strut.

11. The catheter pump as claimed in claim 10, wherein an axial length of the second connecting portion is greater than an axial length of the distal wide body portion and less than an axial length of the distal connecting strut.

12. The catheter pump as claimed in claim 10, wherein an outer wall of the distal connecting portion is provided with a plurality of distal leg grooves, in which the plurality of distal connecting legs are snapped in a one-to-one correspondence, and a distal collar is sleeved over the distal connecting portion and configured to fix the distal connecting legs in a radial direction of the stent so as to retain the distal connecting legs within the distal leg grooves.

13. The catheter pump as claimed in claim 12, wherein
a distal spacing protrusion is formed on the outer wall of the distal connecting portion between two circumferentially adjacent distal leg grooves, the distal spacing protrusion comprises a fourth protruding portion located between two adjacent second connecting portions and a fifth protruding portion located between two adjacent distal connecting struts, and a circumferential width of the fifth protruding portion is greater than a circumferential width of the fourth protruding portion; and/or
the distal collar is made of a metallic material.

14. The catheter pump as claimed in claim 10, wherein
the circumferential width of the distal connecting strut remains unchanged in an extending direction thereof; and/or
the circumferential width of the distal supporting strut remains unchanged in an extending direction thereof; and/or
the circumferential width of the distal wide body portion remains unchanged in the extending direction of the distal connecting strut.

15. The catheter pump as claimed in claim 9, wherein a distal transition portion is provided between the distal supporting strut and the distal connecting strut, and in a direction from a distal end of the distal transition portion to a proximal end of the distal transition portion, a circumferential width of the distal transition portion gradually increases from being equal to that of the distal connecting strut to being equal to that of the distal supporting strut.

16. The catheter pump as claimed in claim 9, wherein the distal connecting portion is a distal bearing chamber connected to a distal end of the stent, a distal bearing is disposed in the distal bearing chamber, and a drive shaft passes through the distal bearing and is connected to the impeller so as to drive the impeller to rotate.

17. The catheter pump as claimed in claim 9, wherein the distal connecting portion is a protective tip connected to a distal end of the distal connecting legs, and the protective tip is flexible so as to separate a blood inlet of the pump head from an inner wall of a ventricle.

18. The catheter pump as claimed in claim 1, wherein a hydrophobic coating is provided on a surface of the stent.
